# EUROPEAN PATENT APPLICATION

(11) **EP 1 424 337 A1**
(43) Date of publication of application: **02.06.2004**
(21) Application number: 02026237.4
(22) Date of filing: 26.11.2002
(51) Int. Cl.: C07D 471/06, A61K 31/437, A61P 1/08

(54) **4-hydroxy derivatives of 5,6,9,10-tetrahydro-10-((2-methyl-1h-imidazol-1-yl)methyl)-4h-pyrido-(3,2,1-jk)-carbazol-11(8h)-one**

(71) Applicant: Solvay Pharmaceuticals GmbH, 30173 Hannover (DE)
(72) Inventor: Brückner, Reinhard, 30559 Hannover (DE); Eeckhout, Christian, 29690 Lindwedel (DE); Finner, Emil, 30916 Isernhagen (DE); Fritsch, Holger, 31515 Wunstorf (DE); Ronken, Eric, 3512 CG Utrecht (NL); Roest, Marco, 1018 AD Amsterdam (NL); Sann, Holger, 30629 Hannover (DE); Schmidt, Jochen, 30173 Hannover (DE); Vandermeij, Paul, 1066 LL Amsterdam (NL)

(57) **Abstract**

The present invention relates to novel 4-hydroxy derivatives of 5,6,9,10-tetrahydro-10-[(2-methyl-1*H*-imidazol-1-yl)methyl]-4*H*-pyrido-[3,2,1-jk]-carbazol-11(8*H*)-one with 5-HT₃-receptor antagonistic activity. Furthermore, the invention relates to a process for the preparation of the novel derivatives.

## Description

The present invention relates to novel 4-hydroxy derivatives of 5,6,9,10-tetrahydro-10-[(2-methyl-1 *H*-imidazol-1-yl)methyl]-4*H*-pyrido-[3,2,1-jk]-carbazol-11(8*H*)-one with 5-HT₃-receptor antagonistic activity. Furthermore, the invention relates to a process for the preparation of the novel derivatives.

5,6,9,10-tetrahydro-10-[(2-methyl-1*H*-imidazol-1-yl)methyl]-4H-pyrido-[3,2,1-jk]-carbazol-11(8*H*)-one derivatives with 5-HT₃-receptor antagonistic properties are known from European Patent EP 0 297 651 B1. One preferred compound which is coming within the scope of disclosure and claims of EP 0 297 651 B1 is (R)-(-)-5,6,9,10-tetrahydro-10-[(2-methyl-1*H*-imidazo)-1-yl)methyl]-4*H*-pyrido-[3,2,1-jk]-carbazol-11(8*H*)-one, which is also known by its INN as cilansetron. Further, cilansetron is also known to be characterized by the chemical name (R)-(-)-4,5,6,8,9,10-hexahydro-10-[(2-methyl-1H-imidazol-1-yl)methyl]-11H-pyrido-[3,2,1-jk]-carbazol-11-one.

The use of, *inter alia,* cilansetron for the production of pharmaceutical preparations for the treatment of functional disturbances in the lower intestinal tracts in larger mammals and humans which involve increased sensitivity to pain and/or abnormally accelerated stool passage in the colon region is already known from European Patent EP 0 601 345 B1.

A process for the preparation of *inter alia* enantiomerically pure cilansetron and an enantiomerically pure cilansetron hydrochloride monohydrate *per* se are known from European patent EP 0 768 309 B1.

5-HT₃-receptor antagonists in general are known to be useful for treating mammals, preferably humans, suffering from or being susceptible to diseases or conditions which can be alleviated or prevented by blockade of the 5 HT₃-receptor. Thus, 5-HT₃-receptor antagonists have been reported to be applicable for a wide range of medical uses, for example for the treatment or prevention of conditions or diseases mediated through 5-HT₃-receptors like functional disturbances of the lower intestines, including visceral pain or Irritable Bowel Syndrome (= IBS), serotonin-induced gastro-intestinal disorders, including emesis induced by anti-cancer agents, migraine, vascular and cluster headache, trigeminal neuralgia, arrhythmia, stress-related psychiatric disorders, depression, cognitive disorders, social withdrawal, panic attacks, agoraphobia, lung embolism, rhinitis or serotonin-induced nasal disorders, for increasing vigilance or for treating dependency induced by dependence-inducing agents.

It has now surprisingly been found, that four novel 4-hydroxy derivatives of 5,6,9,10-tetrahydro-10-[(2-methyl-1*H*-imidazol-1-yl)methyl]-4*H*-pyrido-[3,2,1-jk]-carbazol-11(8*H*)-one according to the invention are themselves active as 5-HT₃-receptor antagonists and that they exhibit a pronounced action component directed at the lower intestines. The novel 4-hydroxy derivatives of 5,6,9,10-tetrahydro-10-[(2-methyl-1*H*-imidazol-1-yl)methyl]-4*H*-pyrido-[3,2,1-jk]-carbazol-11(8*H*)-one appear to be particularly suitable for the treatment and/or prevention of functional disturbances of the lower intestines, including visceral pain or irritable bowel syndrome, and for the treatment and/or prevention of serotonin-induced gastro-intestinal disorders, including emesis induced by anti-cancer agents.

The subject of the invention is therefore novel 4-hydroxy derivatives of 5,6,9,10-tetrahydro-10-[(2-methyl-1*H*-imidazol-1-yl)methyl]-4*H*-pyrido-[3,2,1-jk]-carbazol-11(8*H*)-one of the general formula I, 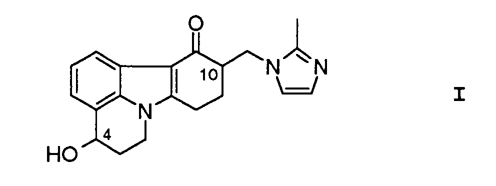 and pharmaceutically acceptable acid addition salts and/or solvates of compounds of formula I.

Preferred compounds of formula I are the compounds of formula la, 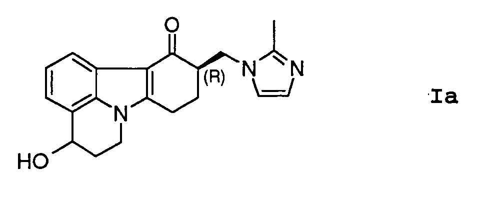 which represent 4-hydroxy derivatives of cilansetron (= 4-hydroxymetabolites) and which are the main metabolites observed in the urine of dogs that had previously been administrated ¹⁴C-labelled cilansetron.

Explicit compounds that are comprised by formula la are the stereoisomers of formula Ib, 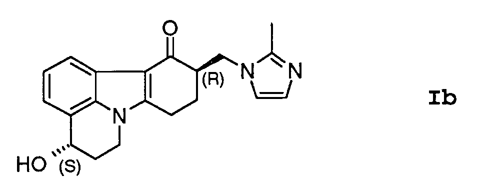 and of formula Ic, 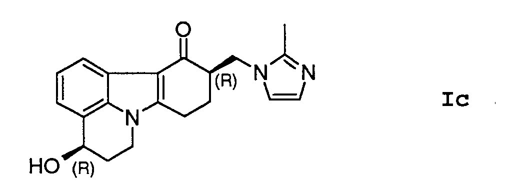 which are both individual embodiments of the invention and which were both individually observed in the urine of dogs that had previously been administrated cilansetron.

Further compounds that represent individual compounds of formula are the compounds of formula Id, 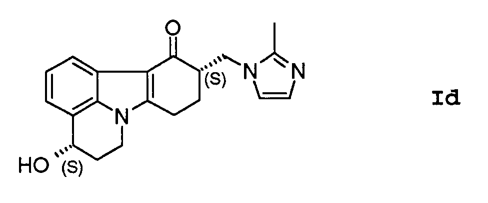 and of formula le,

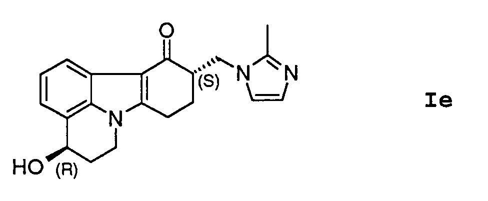

The compounds of formula I and their acid addition salts may be prepared by reacting a compound of general formula II (= mixture of two enantiomers), 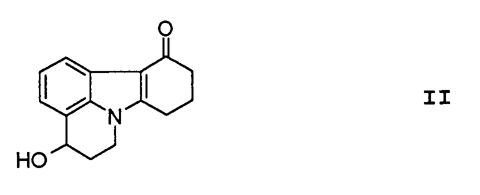 consecutively with 3-oxazolidineethanol and afterwards with 2-methyl-1H-imidazole, optionally followed by separation and isolation of the desired stereoisomers of the compounds of formula I.

In a first step of the preparation process, the compound of formula II is reacted with 3-oxazolidineethanol under acidic reaction conditions. In a second reaction step the resulting intermediate is then reacted with 2-methyl-1H-imidazole. The reaction can be carried out in a known manner as a one-pot reaction in an organic solvent that is inert under the reaction conditions. Suitable solvents are dipolar-aprotic solvents like dimethylformamide (= DMF) or mixtures of such solvents. Suitable reaction temperatures are between room temperature and the boiling point of the solvent or the solvent mixture, but will not exceed 130 °C. Preferable reaction temperatures for the first reaction step are between 80 °C and 100 °C. Preferable reaction temperatures for the second reaction step are between 100 °C and 130 °C. Suitable acids to produce acidic reaction conditions are for example organic acids that are soluble in the solvent used, for example methanesulphonic acid.

The obtained mixture of four stereoisomers Ib, Ic, Id and le may be isolated from the reaction mixture and purified in a known manner. Separation and isolation of the desired isomers of compounds of formula I can be accomplished in a known manner, in particular by performing a High Performance Liquid Chromatography (=HPLC) cascade. Acid addition salts of the separated or unseparated isomers of compounds of formula I may be converted into the free bases in conventional manner, and these may if desired be converted in known manner into pharmaceutically acceptable acid addition salts. Pharmaceutically acceptable acid addition salts of compounds of formula I are their conventional salts with inorganic acids, for example sulphuric acid, phosphoric acids or hydrohalic acids, preferably hydrochloric acid, or with organic acids, for example lower aliphatic monocarboxylic, dicarboxylic or tricarboxylic acids such as maleic acid, fumaric acid, lactic acid, tartaric acid, citric acid, or with sulphonic acids, for example lower alkanesulphonic acids such as methanesulphonic acid or trifluoromethanesulphonic acid, or benzenesulphonic acids optionally substituted in the benzene ring by halogen or lower alkyl, such as p-toluenesulphonic acid.

The compounds of formula II can for example be obtained in a manner as given in the experimental section below.

The compounds of formula I and their acid addition salts and/or solvates have properties which are antagonistic to 5-HT₃-receptors and are therefore suitable for the treatment or prevention of conditions or diseases which are mediated through 5-HT₃ receptors. Due to a a pronounced action component directed at the lower intestines, the compounds of formula I appear to be particularly suitable for the treatment and/or prevention of functional disturbances of the lower intestines, including visceral pain or irritable bowel syndrome, and for the treatment and/or prevention of serotonin-induced gastro-intestinal disorders, including emesis induced by anti-cancer agents. A preferred indication for the compounds of formula I is IBS of mammals, especially humans, of both sexes, in particular non-constipated or diarrhea-predominant IBS.

### Description of the pharmacological test methods

### 1. Affinity of 4-hydroxy derivatives of 5,6,9,10-tetrahydro-10-[(2-methyl-1H-imidazol-1-yl)methyl]-4H pyrido-[3,2,1-jk]-carbazol-11(8H)-one of formulas Ib - le for the human serotonin 5-HT₃ receptor

In this test method, the ability of the 4-hydroxy derivatives of 5,6,9,10-tetrahydro-10-[(2-methyl-1*H*-imidazol-1-yl)methyl]-4*H*-pyrido-[3,2,1-jk]-carbazol-11(8*H*)-one of formulas Ib - le was assessed, for binding to the 5-HT₃ receptor which was endogenously expressed in the human cell line N1E-115 and using tritiated GR-65630 as a radioligand.

The binding assays were carried out using the method of Hoyer and Neijt (see Hoyer, D., Neijt H.C., Mol. Pharmacol. 33, (1988) 303-309). Tritiated GR-65630 was used as radioligand and membrane suspensions, prepared in a known manner from the human neuroblastoma cell line N1E-115. Compounds have been tested for binding in various concentrations to compete with [³H]-GR-65630 (0.35 nM final concentration) in 20 mM HEPES pH 7.4, containing 150 mM NaCl at 25°C for 60 min. Thereafter, the membranes were filtrated over glass fiber filters and bound radioactivity was counted by liquid scintillation counting. Each concentration was tested in triplicate, nonspecific binding was assessed by incubation with the reference compound MDL-72222 (1 µM).

Plotting the amount of bound radioactivity against the concentration of the competing compound allowed estimation of an IC₅₀ value, i.e. that concentration at which 50% of the control radioligand binding remains. This value was subsequently used to calculate the affinity of the compound (pK,) using the equation by Cheng and Prusoff Kₗ= IC₅₀/(1 + S/K_{d}), in which S represents the concentration of the label; Kₗ, is the dissociation constant, which is transformed into a pKₗ by log-transformation; K_{d}, the affinity constant for [³H]-GR-65630 was established separately and found to be 0.33 nM. In this test model, the test substances set forth in Table 1 below exhibited the Kₗ and pKₗ values given below.

**Table 1**

| : Affinity of 4-hydroxy derivatives of 5,6,9,10-tetrahydro-10-[(2-methyl-1 H-imidazol-1-yl)methyl]-4*H*-pyrido-[3,2,1-jk]-carbazol-11(8*H*)-one of formulas Ib - le for the human serotonin 5-HT₃ receptor: | | |
|---|---|---|
| **Compound** | **K**_{**l**} **(nM)** | **pK**_{**l**} |
| Ib | 3.0 | 8.5 |
| Ic | 10 | 8.0 |
| Id | 8.8 | 8.1 |
| le | 38 | 7.4 |

The data show that the 4-hydroxy derivatives of 5,6,9,10-tetrahydro-10-[(2-methyl-1*H*-imidazol-1-yl)methyl]-4*H*-pyrido-[3,2,1-jk]-carbazol-11(8*H*-one of formulas Ib - le exhibit potent displacing activity at the human serotonin 5-HT₃ receptor with pKₗ values that are in the nanomolar range.

### 2. Effect of mammalian cilansetron 4-hydroxymetabolites from dog's urine of formulas Ib and Ic on 2-methyl-5-HT induced contractions in ileal preparations isolated from guinea pigs (5-HT₃ receptor antagonism)

In this test method, the antagonistic potency of the mammalian cilansetron 4-hydroxymetabolites from dog's urine of formulas Ib and Ic was investigated by determining the shift of the concentration-response curve for the specific 5-HT₃ receptor agonist 2-methyl-5-HT in preparations from the guinea-pig ileum.

Guinea-pigs (female, Pirbright White, about 300 g) were killed by a blow on the head and bled from the carotid arteries. lleal segments were isolated and mounted in an organ bath containing a salt solution continuously gassed with carbogen and kept at 37 °C. The composition of the solution in mM was 136.9 NaCl, 2.7 KCI, 1.77 CaCl₂, 0.8 MgCl₂, 0.6 NaH₂PO₄, 11.9 NaHCO₃, and 5 glucose. The preparations were preloaded with 10 mN and allowed to equilibrate for 90 - 120 minutes (= min.). The concentration response curves for 2-methyl-5-HT (0.32 - 100 µM, by company Sigma) were constructed non-cumulatively to avoid desensitization. The measurement was made at the point of maximal contraction and the agonist was washed out as soon as peak response was reached. A first 2-methyl-5-HT curve was followed by another concentration response curve in the presence of one concentration of the antagonist. The response to the highest concentration of 2-methyl-5-HT obtained in the first concentration response curve was set to 100 %. For each compound at least two concentrations of the antagonist were tested in different sets. Data from 5-6 preparations were used for each concentration response curve. Schild plots were calculated and the pA₂ value was determined. The pA₂-value is the negative logarithm of the molar concentration of the antagonist which causes a rightward shift of the concentration response curve for the agonist by a factor of 2. The higher the pA₂-value is, the higher is the potency of a compound.

All test compounds were initially dissolved in dimethylsulfoxide (= DMSO) at concentrations of 10⁻² mol/I and were further diluted with water. The final bath concentrations for the different compounds used were 0.1 and 1 µM for compound of formula Ib and 1 and 3 µM for compound of formula Ic. In this test model, the test substances set forth in Table 2 below exhibited the pA₂-values given below.

**Table 2:**

| Effect of mammalian cilansetron 4-hydroxymetabolites from dog's urine of formulas Ib and Ic on 2-methyl-5-HT induced contractions in ileal preparations isolated from guinea pigs: | |
|---|---|
| **Compound** | **pA**_{**2**} |
| Ib | 6.73 |
| Ic | 6.45 |

In this test method, the 5-HT₃ agonist 2-methyl-5-HT evoked dose-dependent contractions of the guinea-pig ileum with a pD₂-value of 5.2. The pD₂-value is the negative logarithm of the molar concentration response curve to the right. Bath application of mammalian cilansetron 4-hydroxymetabolites from dog's urine of formulas Ib and Ic resulted in a dose-dependent shift of the concentration response curve to the right.

The compounds of formula I may be administered in conventional pharmaceutical preparations. The doses to be used may vary individually and will naturally vary according to the type of condition to be treated. In general, however, medicinal forms with an active substance content of 0.2 to 200 mg, in particular 1 to 50 mg, active substance per individual dose are suitable for administration to humans and larger mammals. The compounds may be contained according to the invention, together with conventional pharmaceutical auxiliaries and/or carriers, in solid or liquid pharmaceutical preparations. Examples of solid preparations are preparations which can be administered orally, such as tablets, coated tablets, capsules, powders or granules, or alternatively suppositories. These preparations may contain conventional pharmaceutical inorganic and/or organic carriers, such as talcum, lactose or starch, in addition to conventional pharmaceutical auxiliaries, for example lubricants or tablet disintegrating agents. Liquid preparations such as suspensions or emulsions of the active substances may contain the usual diluents such as water, oils and/or suspension agents such as polyethylene glycols and the like. Other auxiliaries may additionally be added, such as preservatives, taste correctives and the like.

The active substances may be mixed and formulated with the pharmaceutical auxiliaries and/or carriers in known manner. For the production of solid medicament forms, the active substances may for example be mixed with the auxiliaries and/or carriers in conventional manner and may be wet or dry granulated. The granules or powder can be poured directly into capsules or be pressed into tablet cores in conventional manner. These may be coated in known manner if desired.

### (4S,10R)-4-hydroxy-10-[(2-methy)-1H-imidazol-1-yl)methyl] -5,6,9,10-tetrahydro-4H-pyrido[3,2,1-jk]carbazol-11(8H)-one (compound of formula Ib)

A) 153 g of 1,3-cyclohexanedione was dissolved in a mixture of 765 ml of ethanol and 2296 ml of water. To the formed solution, 199 g of phenylhydrazine, monohydrochloride was added under stirring. When this was dissolved, a total of 110 g of sodium acetate was added portion wise, followed by stirring of the formed suspension for an additional 30 minutes. The precipitate was collected by filtration and washed with water/ethanol (3/1 v/v)) and absolute ethanol. Drying in vacuo at 50°C with a nitrogen flow yielded 243 g 3-(2-phenylhydrazino)-2-cyclohexen-1-one, ¹H-NMR (DMSO/CDCl₃ 4/1): δ=8.7 (NH, broad singlet), 7.74 (NH, broad singlet), 7.1 (2H, m), 6.68 (1H, m), 6.66 (2H, m), 5.03 (1H, broad singlet), 2.37 (2H, t, J=6), 2.1 (2H, t, J=6), 1.85 (2H, quintet, J=6).
B) A solution of 160 g of 3-(2-phenylhydrazino)-2-cyclohexen-1-one obtained above was refluxed for three hours in a mixture of 688 ml of sulfuric acid and 1830 ml of water. The reaction mixture was filtered and the filtrate was poured into 5000 ml of water under cooling with an ice bath. After further cooling to about 5°C, the formed precipitate was filtered off and washed with water. Drying in vacuo at 50°C with a nitrogen flow yielded 88 g of 1,2,3,9-tetrahydro-4H-carbazol-4-one, ¹H-NMR (DMSO/CDCl₃ 4/1): δ=11.75 (NH, broad singlet), 7.97 (1H, m), 7.38 (1H, m), 7.2-7.05 (2H, m), 2.96 (2H, t, J=6), 2.44 (2H, t, J=6), 2.13 (2H, quintet, J=6).
C) 115 g of 1,2,3,9-tetrahydro-4H-carbazol-4-one obtained above under B) and 260 g of potassium carbonate were suspended in 1410 ml of DMF. 75 ml of 2-propenoic acid, ethyl ester was added and the reaction mixture was stirred for 70 hours. The solvent was evaporated and the resulting oil was dissolved in 1750 ml of water. This mixture was extracted four times with 11 of ethyl acetate and the combined ethyl acetate phases were washed with brine and evaporated to dryness. To the resulting oil, 60 g of powdered potassium hydroxide and 1300 ml of absolute ethanol were added. After 70 minutes of refluxing, the solvent was largely evaporated and the resulting solid was dissolved in 700 ml of water. After washing of the aqueous solution with ethyl acetate, 1212 ml of a 1M hydrochloride solution was added. The formed precipitate was collected and washed with ethyl acetate. Drying in vacuo at 50°C with a nitrogen flow yielded 114g 2,3-dihydro-4-oxo-1H-carbazol-9-propanoic acid. ¹H-NMR (DMSO/CDCl3 4/1): δ=12.4 (COOH, broad singlet), 8.03 (1H, m), 7.53 (1H, m), 7.27-7.1 (2H, m), 4.42 (2H, t, J=7), 3.04 (2H, t, J=6), 2.75 (2H, t, J=7), 2.43 (2H, t, J=6), 2.15 (2H, quintet, J=6).
D) 1800 g of polyphosphoric acid was heated to 85°C under stirring. 70 g of 2,3-dihydro-4-oxo-1 H-carbazol-9-propanoic acid obtained above under C) were added and the resulting solution was stirred for 12 hours at 85°C. After cooling to 40°C, 1500 ml of water was added portion wise and the resulting aqueous solution was extracted with dichloromethane. The organic phase was washed consecutively with a 5%-strength aqueous sodium bicarbonate solution and water, dried over magnesium sulfate and evaporated to dryness. Drying of the formed solid in vacuo at 40°C with a nitrogen flow yielded 56 g 5,6,9,10-tetrahydropyrido[3,2,1-jk]carbazol-4,11(8*H*)-dione. ¹H-NMR (CDCl₃): δ=8.36 (1H, dd, J=8, 1.5), 7.73 (1H, dd, J=8, 1.5), 7.33 (1H, t, J=8), 4.42 (2H, t, J=7), 3.1 (2H, t, J=7), 2.98 (2H, t, J=6), 2.6 (2H, t, J=6), 2.3 (2H, quintet, J=6).
E) A suspension of 79 g of 5,6,9,10-tetrahydropyrido[3,2,1-jk]carbazol-4,11(8*H*)-dione obtained above under D) in 500 ml of dichloromethane and 500 ml of methanol was cooled to 0°C. 15 g of sodium borohydride was added thereto in small portions. The temperature was allowed to rise and the mixture was stirred for 50 minutes at ambient temperature. Subsequently, 54 ml of a 35 %-strength aqueous hydrogen peroxide solution and 27 ml of 2N aqueous sodium hydroxide solution were added and the resulting suspension was stirred for an additional 20 minutes. The mixture was extracted with dichloromethane, the dichloromethane phase was separated and washed with brine, dried over sodium sulphate and evaporated to dryness. Drying in vacuo at 50°C with a nitrogen flow yielded 77 g 4-hydroxy-5,6,9,10-tetrahydro-4H-pyrido[3,2,1-jk]carbazol-11(8*H*)-one (compound of formula II), ¹H-NMR (DMSO/CDCl₃ 4/1): δ=7.8 (1H, dd, J=7, 2), 7.2-7.05 (2H, m), 5.44 (OH, d, J=5), 4.95 (1H, quartet, J=5), 4.16 (2H, m), 2.98 (2H, t, J=6), 2.44 (2H, m), 2.25-2.05 (4H, m).
F) A mixture of 72 g of 4-hydroxy-5,6,9,10-tetrahydro-4H-pyrido[3,2,1-jk]carbazol-11 (8*H*)-one obtained above under E), 90 g of methanesulphonic acid and 700 ml of DMF was heated to 85°C. To the formed solution, 109 g of 3-oxazolidineethanol was added and the reaction mixture was stirred for 4.5 hours at 100°C. After addition of 197 g of 2-methyl-1H-imidazole, the mixture was stirred for 1.5 hours at 120°C. The solvent was then evaporated, the residue taken up in water and extracted with dichloromethane. The organic phase was washed with water and concentrated. Chromatography of the residue on silica gel (mobile solvent: dichloromethane and methanol/dichloromethane (10/90 % v/v), yielded 24 g 4-hydroxy-5,6,9,10-tetrahydro-10-[(2-methyl-1*H*-imidazol-1-yl)methyl]-4*H*-pyrido-[3,2,1-jk]-carbazol-11 (8*H*)-one,¹H-NMR (DMSO/CDCl₃ 4/1 v/v): δ=7.83 (1H, m), 7.25-7.1 (2H, m), 7.02 (1H, d, J=1.5), 6.73 (1H, d, J=1.5), 5.49 (OH, d, J=5), 4.94 (1H, q, J=5), 4.46 (1 H, dd, J=14, 5), 4.25-4.0 (3H, m), 3.2-2.8 (3H, m), 2.34 (3H, s), 2.18 (2H, q, J=5), 2.1 (1H, m), 1.82 (1H, m).
G) The four possible isomers of the 4-hydroxy-5,6,9,10-tetrahydro-10-[(2-methyl-1*H*-imidazol-1-yl)methyl]-4*H*-pyrido-[3,2,1-jk]-carbazol-11(8*H*-one obtained above under F) were separated by an HPLC-cascade. First the diastereomeric pairs were separated on an inertsil ODS-3, 8 µm column. The mobile phase of this separation consisted of a mixture of acetonitrile and water containing 0.1 % of formic acid with the acetronitrile content increasing from 8 to 90% during the elution. Each diastereomeric pair (pair A: 4S-10R, compound of formula Ib, and 4R-10S (compound of formula le), pair B: 4R-10R (compound of formula Ic) and 4S-10S (compound of formula Id) was then chromatographed on a chiracel OK® , 20 µm column with a mobile phase of methanol containing 0.1 % of diethylamine. Then all isomers were again subjected to diastereomeric HPLC to remove some diastereomeric impurity.
   The enantiomers were thus received from chromatography in the following order:
   Isomer (1): compound of formula Ib (isomer 4S-10R)
   Isomer (2): compound of formula Id (isomer 4S-10S)
   Isomer (3): compound of formula Ic (isomer 4R-10R)
   Isomer (4): compound of formula le (isomer 4R-10S).
H) To transform the formic acid salt of the enantiomeric pure compound of formula Ib as directly obtained from the chromatographic separation step above into the respective hydrochloric acid hydrate, 22 g of the formic acid salt of compound of formula Ib was dissolved in a mixture of 10 ml of demineralised water and 5 ml of 1-butanol. 10 ml of concentrated hydrochloric acid was added to that receiving solution and the resulting suspension was diluted with 125 ml of 1-butanol and cooled to 0°C. After 90 min. of stirring at 0°C, crystals had precipitated and were collected, consecutively washed with 1-butanol and methyl-tert.-butyl ether (= MTBE) and dried in vacuo at 50°C with a nitrogen flow to yield 21 g (4S,10R)-4-hydroxy-10-[(2-methyl-1H-imidazol-1-yl)methyl]-5,6,9,10-tetrahydro-4H-pyrido[3,2,1-jk]carbazol-11(8H)-one-hydrochloride monohydrate.

To assign absolute configurations of the isomers, a single crystal was grown of the hydochloride monohydrate of isomer (1) *(vide supra):* 3,3 mg of isomer (1) were dissolved stepwise in 1.5 ml of acetonitrile at 50°C in an oil bath by adding one drop of demineralized water. Crystallization was achieved by slowly cooling to room temperature followed by storing the solution in a refrigerator at approximately 6 °C. The absolute configuration was then determined by single-crystal X-ray diffraction analysis. The crystal data of the single crystal of isomer (1) is given below in table 3:

**Table 3:**

| Crystal data of single crystal from the hydochloride monohydrate of isomer (1): | |
|---|---|
| empiric formula | C₂₀H₂₄ClN₃O₃ |
| temperature | 143(2) K |
| wavelength | 0.71073 Å |
| crystal system | monoclinic |
| space group | P2₁ |
| unit cell dimensions | a = 15.3703(14) Å α = 90° b = 7.4994(8) Å β= 92.117(3)° c = 16.2987(14) Å γ= 90° |
| volume | 1877.4(3) Å |
| Z | 4 |
| density (calculated) | 1.379 Mg/m³ |
| crystal size | 0.50 x 0.35 x 0.14 mm³ |
| final R indices [I>2sigma(I)] | R1 = 0.0302, wR2 = 0.0795 |
| R indices (all data) | R1 = 0.0337, wR2 = 0.0814 |
| absolute structure parameter | -0.03(3) |
| largest difference peak and hole | 0.324 and -0.234 e. Å³ |

The absolute stereochemistries at the chiral centers of isomer (1) were thus proven to be 4S and 10R.

The absolute stereochemistries at the chiral centers of isomer (4) could then be deduced to be 4R and 10S, due to the fact that isomer (4) must be the enantiomer of isomer (1), as determined by achiral HPLC and NMR experiments.

Isomer (3) had been identified as the second main metabolite from dog's urine by chiral HPLC-UV and NMR. Since optical pure cilansetron of 10R configuration had been administrated to the dogs and since no racemisation at C(10) of the parent cyclic structure of cilansetron takes place during metabolism in the dog, the absolute stereochemistries at the chiral centers of isomer (3) could be concluded to be 4R and 10R. Furthermore, isomer (3) had been identified as diastereomer of isomer (1) by e.g. achiral HPLC experiments.

The absolute stereochemistries at the chiral centers of isomer (2) could then be deduced to be 4S and 10S, due to the fact that isomer (2) must be the enantiomer of isomer (3), as determined by achiral HPLC and NMR experiments.

The absolute configurations of isomers (1) - (4) could also be assigned by racemisation experiments known *perse.*

### Example I:

Capsules containing (4S,10R)-4-hydroxy-10-[(2-methyl-1H-imidazol-1-yl)methyl]-5,6,9,10-tetrahydro-4H-pyrido[3,2,1-jk]carbazol-1(8H)-one-hydrochloric acid hydrate:

Capsules with the following composition per capsule were produced:

| | |
|---|---|
| (4S, 10R)-4-hydroxy-10-[(2-methyl-1H-imidazol-1-yl)methyl]-5,6,9,10-tetrahydro-4H-pyrido[3,2,1-jk]carbazol-1 (8H)-one-hydrochloric acid hydrate | 20 mg |
| Corn starch | 60 mg |
| Lactose | 300 mg |
| Ethyl acetate (EA) | q.s. |

The active substance, the corn starch and the lactose were processed into a homogenous pasty mixture using EA. The paste was ground and the resulting granules were placed on a suitable tray and dried at 45°C in order to remove the solvent. The dried granules were passed through a crusher and mixed in a mixer with the further following auxiliaries:

| | |
|---|---|
| Talcum | 5 mg |
| Magnesium stearate | 5 mg |
| Corn starch | 9 mg |

and then poured into 400 mg capsules (= capsule size 0).

## Claims

1. A compound of the general formula I, 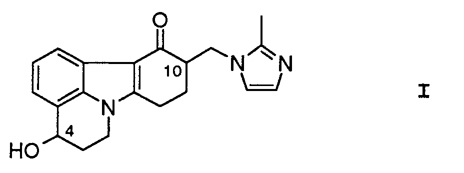 or a pharmaceutically acceptable acid addition salt and/or a solvate of a compound of formula I.

2. A compound of formula la, 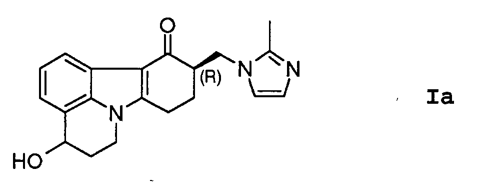 according to claim 1.

3. A compound of formula Ib, 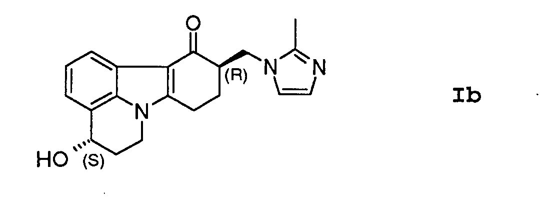 according to claim 1.

4. A compound of formula Ib according to claim 3 in the form of its hydrochloride monohydrate.

5. A compound of formula Ib according to claim 4, wherein the hydrochloride monohydrate is crystalline.

6. A compound of formula Ic, 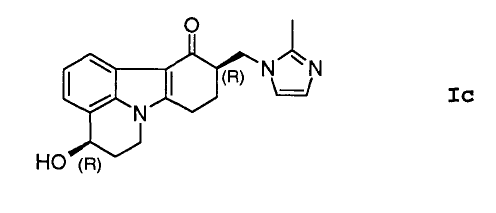 according to claim 1.

7. A pharmaceutical composition comprising a pharmacologically effective quantity of a compound according to claim 1 and conventional pharmaceutical auxiliaries and/or carriers.

8. A method for the blockade of 5-HT₃ receptors in a mammal which comprises the administration to the mammal of a compound of claim 1 in an amount that is effective for antagonizing the effect of serotonin at its 5-HT₃ receptor site.

9. The use of a compound according to claim 1 as a 5-HT₃-receptor antagonist.

10. The use of a compound according to claim 1 for the manufacture of a medicament for the treatment or prevention of a condition or disease mediated through 5-HT₃ receptors.

11. The use according to claim 9, wherein the condition or disease is selected from a functional disturbance of the lower intestines and from serotonin-induced gastro-intestinal disorders, including emesis.

12. The use according to claim 11, wherein the functional gastrointestinal disturbance of the lower intestines is associated with increased pain sensitivity upon stool passage through the lower intestine, visceral pain and/or anomalies in stool passage through the lower intestine.

13. The use according to claim 12, wherein the functional gastrointestinal disturbance of the lower intestines is Irritable Bowel Syndrome.

14. The use according to claim 13, wherein the functional gastrointestinal disturbance of the lower intestines is non-constipated or diarrhea-predominant Irritable Bowel Syndrome.

15. A process for the preparation of compounds of the general formula 1, 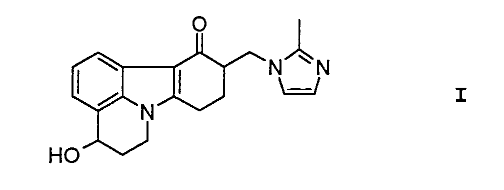 **characterised in that** a compound of the general formula 11, 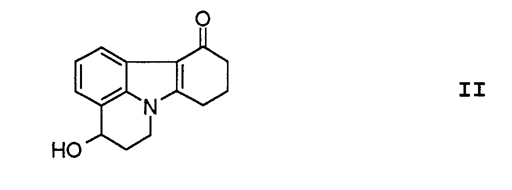 is reacted consecutively with 3-oxazolidineethanol and afterwards with 2-methyl-1 H-imidazole, optionally followed by separation and isolation of the desired stereoisomers of the compounds of formula I.
